# EUROPEAN PATENT APPLICATION

(11) **EP 3 466 434 A1**
(43) Date of publication of application: **10.04.2019**
(21) Application number: 16902691.1
(22) Date of filing: 26.05.2016
(51) Int. Cl.: A61K 33/26, A61K 31/198, A61P 3/02

(54) **USE OF COMPOSITION COMPRISING FERROUS AMINO ACID CHELATE FOR MANUFACTURE OF MEDICINE FOR REDUCING LACTIC ACID**

(71) Applicant: Profeat Biotechnology Co. Ltd., Taoyuan City 33045 (TW)
(72) Inventor: LIN, Tsun-Yuan, Taoyuan City (TW); CHEN, Mu-Kuei, Taoyuan City (TW); CHEN, Tsang-Tse, Taoyuan City (TW); FU, Chai-Hui, Taoyuan City (TW); JAN, Hsun-Jin, Taoyuan City (TW)
(74) Representative: Cabinet Chaillot
(86) International application number: PCT/CN2016/083400
(87) International publication number: WO 2017/201701

(57) **Abstract**

Disclosed is a use of a composition comprising a ferrous amino acid chelate for manufacture of a medicine for reducing lactic acid. The medicine comprises a therapeutically effective amount of a ferrous amino acid chelate-containing composition and a pharmaceutically acceptable carrier thereof. The ferrous amino acid chelate-containing composition can effectively reduce the accumulation of lactic acid in cancer cells, reduce the uptake amount of glucose, increase the motility, increase the lactate dehydrogenase, and effectively prevent and treat the related symptoms caused by exercise fatigue.

## Description

### Field of the Invention

The present invention relates to a use of a composition containing a ferrous amino acid chelate, especially to a use of the composition for the preparation of pharmaceuticals used in reduction of lactic acid.

### Description of the prior art

Carbohydrates are the main source of energy used in sports, and the way to produces energy is divided into aerobic and anaerobic. Carbohydrates decompose into lactic acid and produce energy under anaerobic conditions, wherein the lactic acid is converted from pyruvate by lactate dehydrogenase. When the exercise intensity is low, the energy generated from carbohydrates is mainly under the aerobic condition, and the anaerobic participation is less. Therefore, the rate of lactic acid production is not high, and then lactic acid can be easily metabolized and would not be accumulated in the body and blood. When the exercise intensity becomes more intense, the energy generated from carbohydrates will be converted to mainly anaerobic condition and the lactic acid production will also become quicker. Once the metabolization of the lactic acid cannot catch up with the production of the lactic acid, the lactic acid will be accumulated in the body and blood.

The fatigue caused by excessive exercise is called exercise fatigue. When oxygen and nutrients continue to be consumed and lactic acid continues to be accumulated, the muscles become stiff due to a decrease in contractile function, and muscle fibers may tear due to severe contraction, causing discomfort such as muscle soreness and muscle weakness. The drugs commonly used in convention for treating exercise fatigue are usually accompanied by varying degrees of side effects. For example, muscle relaxants may cause side effects such as diarrhea, dry mouth, and drowsiness. Non-steroidal anti-inflammatory drugs may cause digestive tract mucosal damage, liver dysfunction, dizziness, headache, drowsiness and other side effects.

Taiwan Patent Publication No. 201406381 discloses a pharmaceutical composition for anti-exercise fatigue, which is a rutin for effectively reducing the amount of lactic acid in the blood and for treating related symptoms caused by exercise fatigue. However, the side effects that rutin may cause are allergies, tight throat or chest, and breathing disorders.

Therefore, how to develop a drug which can be used for against exercise fatigue, has low side effects, and can effectively reduce the amount of lactic acid accumulation to effectively prevent and treat related symptoms caused by exercise fatigue; the prior art needs to be improved.

### Summary of the invention

In view of the disadvantages of prior art drug side effects, the object of the present invention is to provide a use of a composition containing a ferrous amino acid chelate for the preparation of pharmaceuticals used in reducing lactic acid, wherein the composition containing the ferrous amino acid chelate has an effect on regulation of lactic acid metabolism.

To achieve the above objective, the objective of the present invention is to provide the use of the composition containing a ferrous amino acid chelate for preparation of pharmaceuticals used in reducing lactic acid, wherein the pharmaceuticals comprise an effective amount of the composition containing the ferrous amino acid chelate and pharmaceutically acceptable carriers.

According to the present invention, the term "the composition containing the ferrous amino acid chelate" refers to the composition made by mixing an inorganic iron with an amino acid.

Preferably, the chelating ratio of the ferrous to the amino acid of the composition containing the ferrous amino acid chelate is between 1:1 and 1:4.

More preferably, the chelating ratio of the ferrous to the amino acid of the composition containing the ferrous amino acid chelate is between 1:1.5 and 1:2.5.

Preferably, the effective amount of the composition containing the ferrous amino acid chelate is between 0.1 mg/kg/day and 5 mg/kg/day.

More preferably, the effective amount is between 0.5 mg/kg/day and 3 mg/kg/day.

More preferably, the effective amount is between 0.65 mg/kg/day and 2.93 mg/kg/day.

According to the announcement of Food and Drug Administration of estimating the maximum safe starting dose in initial clinical trials for therapeutics in adult healthy volunteers in 2005, the daily recommended dosage is converted in principle to 12.3 times per kilogram base on 60 kg adults body weight for a one-time dosage of mouse. Therefore, in the case of administration of the mouse from about 8 mg/g/day to 36 mg/g/day, the conversion to the human body is about 0.65 mg/kg/day to 2.93 mg kg/day.

Preferably, the effective amount of the composition containing the ferrous amino acid chelate for mouse is between 8 mg/g/day and 36 mg/g/day.

More preferably, the composition containing the ferrous amino acid chelate is prepared from mixing ferrous sulfate with glycine followed by heating between 60°C and 90°C for 8 hours to 48 hours, wherein a weight ratio of the ferrous sulfate to the glycine of ferrous amino acid chelate is between 1:1.2 and 1:1.5.

Preferably, the inorganic iron is ferrous sulfate, ferrous chloride, ferrous pyrophosphate or combinations thereof; the amino acid is glycine.

More preferably, the composition containing the ferrous amino acid chelate comprises 95% to 100% weight percentage of the ferrous glycine chelate. Furthermore preferably, the composition containing the ferrous amino acid chelate comprises 98% to 99.9% weight percentage of the ferrous glycine chelate.

Preferably, the composition containing the ferrous amino acid chelate comprises a reducing agent. The reducing agent comprises, but is not limited to ascorbic acid, citric acid, acetic acid, propionic acid, butyric acid, lactic acid, malic acid, sulfonic acid, succinic acid or combinations thereof.

The term "effective amount" as used herein, refers to a dosage to reduce lactic acid in a period of time. According to the present invention, the effective amount is determined for reducing accumulation amount of the lactic acid or reducing the oxidative pressure of lung cancer, breast cancer or brain cancer cells by administering the composition containing ferrous amino acid chelate in a specific amount. According to the present invention, it can also mean that increase activity and reduce the accumulation of lactic acid.

According to the present invention, the term " pharmaceutically acceptable carriers" includes, but is not limited to solvent, emulsifier, suspending agent, decomposer, binding agent, excipient, stabilizing agent, chelating agent, diluent, gelling agent, preservative, lubricant, surfactant, and the like, or other suitable carriers of the present invention.

The pharmaceuticals in accordance with the present invention comprise various dosage forms, and the dosage form comprises, but is not limited to liquid, semi-solid and solid. The liquid comprises, but is not limited to solution, emulsion, suspension, powder, tablet, pill, lozenge, troche, chewing gum, slurry, liposome, suppository or other suitable dosage forms of the present invention.

Preferably, the mode of the pharmaceuticals in accordance with the present invention is an enteral administration or parenteral administration.

More preferably, the mode of enteral administration is oral dosage. The oral dosage form is solution, emulsion, suspension, powders, tablet, pill, lozenge, troche, capsules or chewing gum.

The advantages of the invention are as follows.
1. The composition containing a ferrous amino acid chelate of the present invention is confirmed that it can effectively reduce the accumulation of lactic acid of cancer cells and reduce glucose uptake by administration.
2. The administration of the composition containing a ferrous amino acid chelate to a healthy receptor can effectively increase the activity, reduce the accumulation of lactic acid, and increase the efficacy of lactate dehydrogenase.

### In The Drawings

The present disclosure will become more fully understood from the detailed description and the accompanying drawings, in which:
Fig. 1 shows a bar chart related to the accumulation of lactic acid of a group administering the composition containing ferrous amino acid chelate (hereinafter referred to as X) to non-small cell lung cancer cell A549.
Fig. 2 shows a bar chart related to accumulation of lactic acid of a group administering the composition X to non-small cell lung cancer cell H460.
Fig. 3 shows a bar chart related to accumulation of lactic acid of a group administering the composition X to breast cancer cells MDA-MB-231.
Fig. 4 shows a bar chart related to accumulation of lactic acid of a group administering the composition X to glioma cells U87-MG.
Fig. 5 shows a bar chart related to the protein expression of hypoxia inducible factor-1α (HIF-Iα) of non-small cell lung cancer cell A549 administrating the composition X under normal or hypoxic state, wherein β-actin as a reference. Each group was repeated three times, and * indicates that compared with the control group, p<0.05, # indicates that compared with only cobalt chloride (CoCl₂) administration p<0.05.
Fig. 6 is a bar chart showing the percentage of glucose uptake compared to the control group after the composition X administration on brain glioma cell line U87-MG. * indicates p<0.05 compared to the control group.
Fig. 7 is a distribution plot of forced swimming test time after feeding the composition X to mice of the institute of cancer research (ICR).
Fig. 8 is a bar chart of serum lactate concentration (mg/dL) in ICR mice after the composition X administration.
Fig.9 is a bar chart of serum lactate dehydrogenase (LDH) concentrations (IU/L) in ICR mice after the composition X administration.

Other objectives, advantages and novel features of the invention will become more apparent from the following detailed description when taken in conjunction with the accompanying drawings.

### Preparation example 1: Preparation of the composition containing ferrous amino acid chelate

The composition containing ferrous amino acid chelate was prepared as follows. Firstly, ferrous sulfate and glycine (more than 98% purity) were mixed in a weight ratio of 1: 1.3 through 60°C to 90°C and heated for 8 hours to 48 hours to obtain the composition comprising ferrous amino acid chelate, wherein the chelating ratio of ferrous and amino acid of the ferrous amino acid chelate was between 1: 1 and 1: 4, and the concentration of the composition comprising ferrous amino acid chelate was modulated to 5 µg/ml, 10 µg/ml, 25 µg/ml, 30 µg/ml, 50 µg/ml, 100 µg/ml, 250 µg/ml, 500 µg/ml or 1000 µg/ml, and the composition referred to as X.

### Preparation example 2: Culture of lung cancer cell

A549 AND H460 cells were respectively seeded in DMEM medium containing 10% fetal bovine (FBS), 1% penicillin (100U/mL)-streptomycin (100 g/ml), and 1% glutamine (200 mM) under 37 °C and 5% CO₂ about 70% to 80% cell adhesion for subculture.

### Preparation example 3: Culture of breast cancer cell

MDA-MB-231 breast cancer cells were seeded in DMEM medium containing 10% fetal bovine (FBS), 1% penicillin (100U/mL)-streptomycin (100 g/ml), and 1% glutamine (200 mM) under 37 °C and 5% CO₂ about 70% to 80% cell adhesion for subculture.

### Preparation example 4: Culture of brain cancer cell

U87-MG glioma cells were seeded in RPMI medium containing 10% fetal bovine (FBS), 1% non-essential amino acid (NEAA), and 1% penicillin (100U/mL)-streptomycin (100 g/ml) under 37 °C and 5% CO₂ for 3-4 days subculture once.

### Example 1 Expression of lactic acid secretion by cancer cells treated with composition X

The cancer cells (lung cancer, breast cancer, and brain cancer) cultured in preparation examples 2 to 4 were seeded into a cell well plate at 3×10⁴ cells per well, and then 30 µg/ml, 100 µg/ml, 250 µg/ml, 500 µg/ml composition X prepared in preparation example 1 were added to per well to treat three cancer cells respectively, wherein no composition X adding as a control group. After 24 hours, the cell supernatant was collected and the accumulation amount of lactic acid secreted from the cancer cells in the cell culture solution was measured using an L-lactate assay kit (abeam® ab65331).

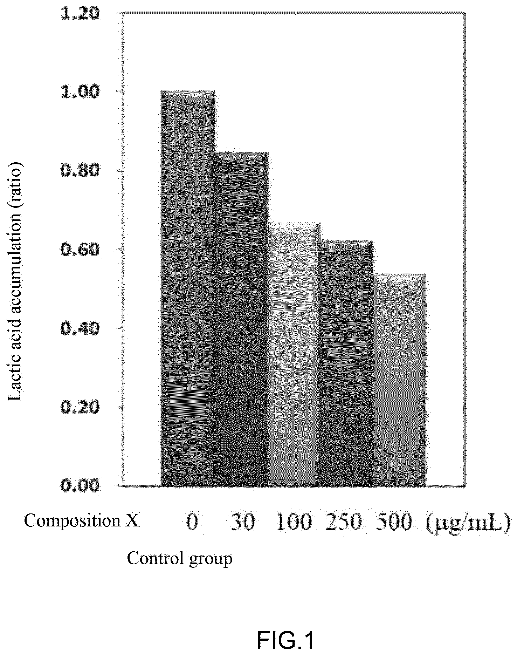

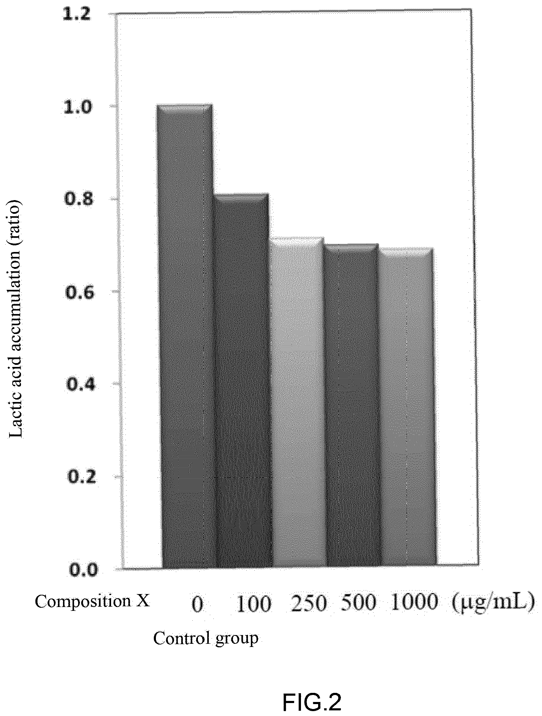

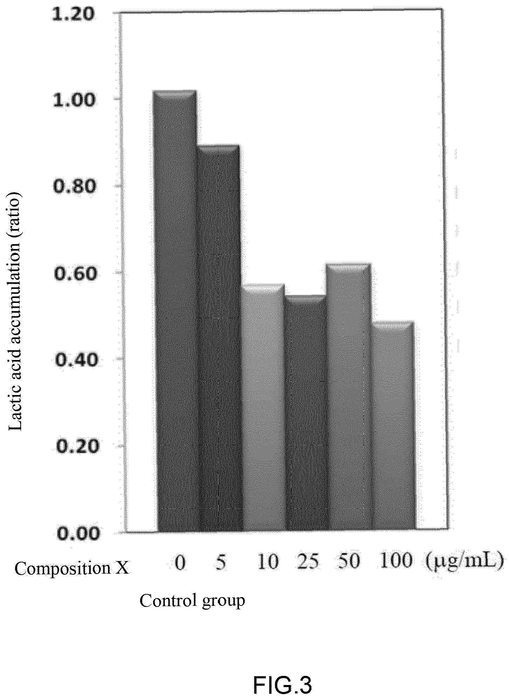

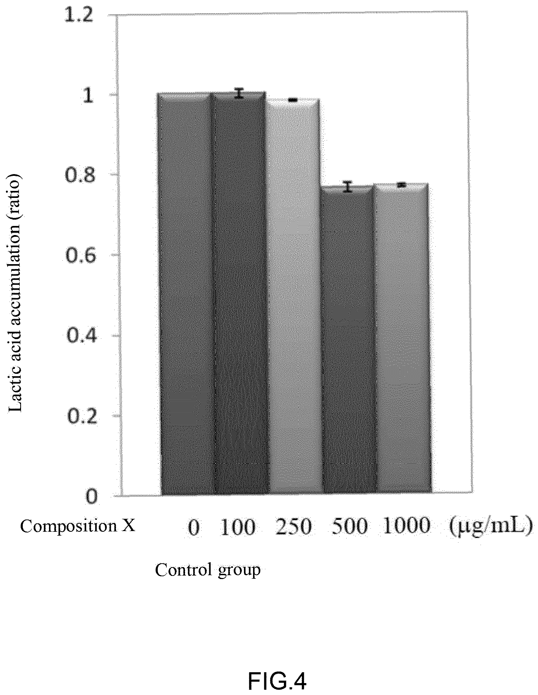

As shown in Fig. 1, when 30 µg/ml composition X was administered in the non-small cell lung cancer cell line A549, the accumulation amount of lactic acid was reduced by about 20% compared with the control group. When the concentration of substance X increased, the accumulation amount of lactic acid was decreased by nearly 50% in the group administered with the 500 µg/ml composition X. As shown in Fig. 2, when 100 µg/mL composition X was administered into the non-small cell lung cancer cell line H460, the accumulation amount of lactic acid was reduced by about 20% compared with the control group. While the concentration of the composition X was increased from 250 µg/ml to 1000 µg/ml, and the accumulation amount of lactic acid decreased to about 30%. As shown in Fig. 3, when 5 µg/ml composition X was administered to the breast cancer cell MDA-MB-231, the accumulation amount of lactic acid was reduced by about 10% compared with the control group. While the concentration of composition X was increased from 10 µg/ml to 100 µg/ml, the accumulation amount of lactic acid was reduced by about 40% to 50%. As shown in Fig. 4, when the concentration of composition X was from 500 µg/ml to 1000 µg/ml administered to the brain glioma cell line U87-MG, the accumulation amount of lactic acid was decreased about 20% compared with the control group.

### Example 2 Expression of HIF-1α after treatment of composition X with lung cancer cells under normal or hypoxia state

The non-small cell lung cancer cell line A549 cultured in the preparation example 2 was seeded into the cell well plate at 3×10⁴ cells per well, and the 100 µg/ml or 250 µg/ml composition X prepared in the preparation example 1 was respectively administered to non-small cell lung cancer cells A549 under normal oxygen state; the group which no composition X added was a control group. After 24 hours, western blotting was used to analyze the protein expression of HIF-1α. In addition, in the case of hypoxia induced by CoCl₂, the non-small cell lung cancer cell A549 was first treated with 200 µM CoCl₂for 3 hours, and then the 100 µg/ml or 250 µg/ml composition X was administered for 24 hours. The protein expression of HIF-1α was quantitative analyzed based on β-actin by western blotting.

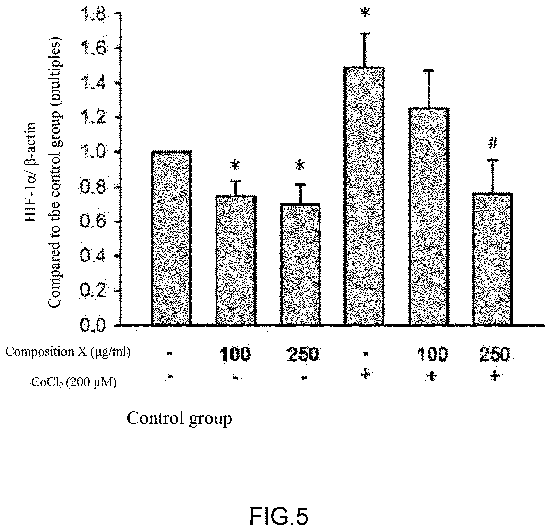

As shown in Fig. 5, when 100 µg/ml or 250 µg/ml composition X was respectively administered to the non-small cell lung cancer cell A549 in the normal oxygen state, the protein expression of HIF-1α was significantly decreased more than 20% compared to the control group. In hypoxia state, the protein expression of HIF-1α in the untreated composition X group was significantly higher than that in the control group, whereas the protein expression level of HIF-1α of the group administered 100 µg/ml composition X was lower than that in the untreated composition X group. When the concentration of the composition X was 250 µg/ml, the protein expression of HIF-1α was significantly lower than that of the untreated composition X group, and the concentration of HIF-1α was even similar to the concentration of 100 µg/ml or 250 µg/ml composition X administered under normal oxygen state. Thus, the composition of the present invention can reduce HIF-1α production under normal oxygen state or hypoxic state for non-small cell lung cancer cells line A549.

### Example 3 Composition X inhibits glucose uptake by brain glioma cells

The human glioma cells U87-MG prepared in the preparation example 1 were seeded into the cell well plate at 3×10⁴ cells per well, and 0 µg/ml (control group), 100 µg/ml (200 µM CoCl₂ was added at first 3 hours to simulate hypoxia), 30 µg/ml, 100 µg/ml composition X was treated for 24 hours respectively. After Krebs-Ringer buffer (KRB) for starvation 30 minutes, glucose containing labeled radioactive elements ³H was added for 3 hours, and then wash 3 times with PBS. Cells were collected with lysis buffer, and then put into in scintillation liquid and measured by b-counter.

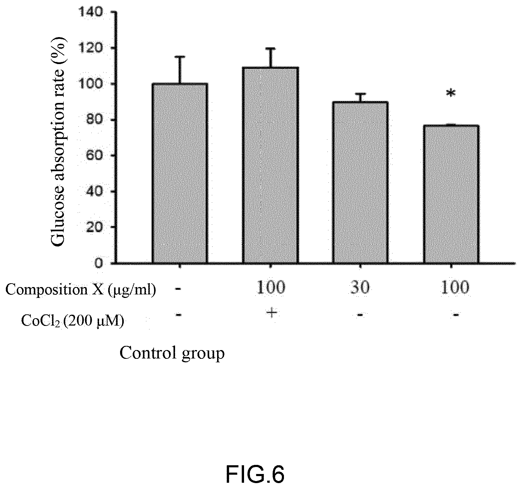

As shown in Fig. 6, when 100 µg/ml composition X treated brain glioma cells in the hypoxic state, the glucose uptake was not significantly different from that of the control group.

When 30 µg/ml composition X was administered to brain glioma cells, the glucose uptake was decreased compared with the control group in the oxygen state. When the concentration of the composition X was 100 µg/ml, the glucose uptake was significantly reduced more than 20% compared to the control group.

### Example 4 Effect of the composition X on activity of experimental animals

For the American Institute of Cancer research (ICR) mice (each weighing about 25 grams), the compositions X prepared in preparation example 1 were fed with 0 µg (as a control group), 200 µg, and 900 µg per day respectively, and at least 7 mice in each group were subjected to experiments.. After two weeks of continuous feeding, a forced swimming test was performed.

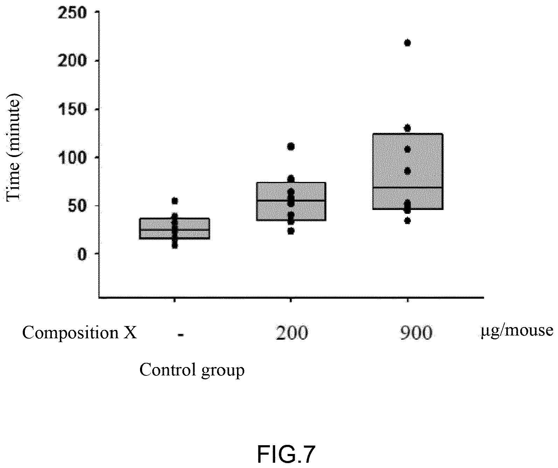

As shown in Fig. 7, mice fed 200 µg or 900µg composition X per day had an increasing trend in average swimming time compared to the control group and a positive correlation with increased feeding.

### Example 5 Effect of the composition X on lactic acid accumulation in serum of experimental animals

For each of the ICR mice, the composition X prepared in preparation example 1 was fed with 0 µg (as a control group), 200 µg and 900 µg per day respectively, and at least 7 mice in each group were subjected to experiments. After two weeks of continuous feeding, blood of mice was taken to test the content of lactic acid and lactate dehydrogenase in the serum after the forced swimming test.

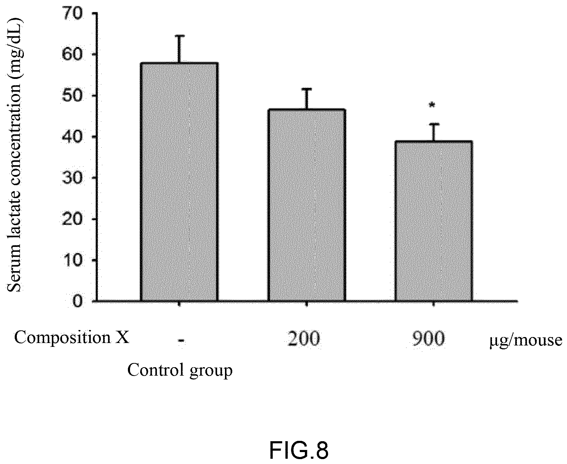

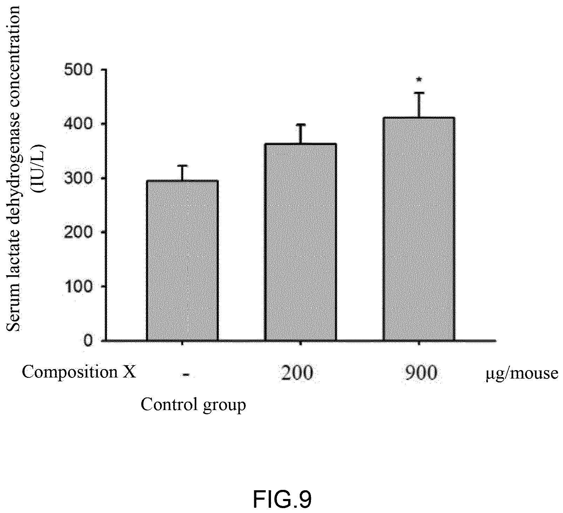

As shown in Fig. 8, mice fed 200 µg composition X per day had a trend toward a decrease in serum lactic acid content compared to the control group. While the mice fed 900 µg composition X per day, the content of lactic acid in serum was significantly reduced compared to the control group. As shown in Fig. 9, compared to the control group, mice fed 200 µg composition X per day had an increased tendency to lactate dehydrogenase in serum. While the mice fed 900 µg composition X per day, the content lactate dehydrogenase in serum was significantly increased compared to the control group.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its spirit and scope.

## Claims

1. A use of a composition contains a ferrous amino acid chelate for the preparation of pharmaceuticals used in reducing lactic acid, wherein the pharmaceuticals comprise an effective amount of the composition containing the ferrous amino acid chelate and pharmaceutically acceptable carriers.

2. The use as claimed in claim 1, wherein the chelating ratio of ferrous iron to the amino acid of the composition containing the ferrous amino acid chelate is between 1:1 and 1:4.

3. The use as claimed in claim 1, wherein the chelating ratio of ferrous iron to the amino acid of the composition containing the ferrous amino acid chelate is between 1:1.5 and 1:2.5.

4. The use as claimed in claim 1, wherein the effective amount of the composition containing the ferrous amino acid chelate is between 0.1 mg/kg/day and 5 mg/kg/day.

5. The use as claimed in claim 1, wherein the effective amount of the composition containing the ferrous amino acid chelate is between 0.5 mg/kg/day and 3 mg/kg/day.

6. The use as claimed in any one of claims 1 to 5, wherein the composition containing the ferrous amino acid chelate is prepared from mixing an inorganic iron with an amino acid followed by heating between 60°C and 90°C for 8 hours to 48 hours, wherein the ratio of the inorganic iron to the amino acid is between 1:1.2 and 1:1.5.

7. The use as claimed in claim 6, wherein the inorganic iron is ferrous sulfate, ferrous chloride, ferrous pyrophosphate or combinations thereof, wherein the amino acid is glycine.

8. The use as claimed in claim 6, wherein the composition containing the ferrous amino acid chelate comprises a reducing agent, wherein the reducing agent comprises ascorbic acid, citric acid, acetic acid, propionic acid, butyric acid, lactic acid, malic acid, sulfonic acid, succinic acid or combinations thereof.

9. The use as claimed in claim 1, wherein a dosage form of the pharmaceuticals is for enteral administration or parenteral administration.

10. The use as claimed in claim 9, wherein the dosage form of the pharmaceuticals for enteral administration is oral dosage form, wherein the oral dosage form comprises solution, emulsion, suspension, powder, tablet, pill, lozenge, troche, chewing gum, or capsule.
